# EUROPEAN PATENT SPECIFICATION

(11) **EP 3 329 800 B1**
(45) Date of publication and mention of the grant of the patent: **02.09.2020**
(21) Application number: 16830286.7
(22) Date of filing: 11.07.2016
(51) Int. Cl.: A45D 34/04, A61K 8/02, A61Q 1/02, D04H 1/541, B32B 27/12, B32B 5/02

(54) **PAD FOR HOLDING/SUPPLYING LIQUID COSMETICS**
PAD ZUM HALTEN/ZUFÜHREN VON FLÜSSIGER KOSMETIK
TAMPON DESTINÉ À CONTENIR/DISTRIBUER DES PRODUITS COSMÉTIQUES LIQUIDES

(30) Priority: 30.07.2015 JP 2015150332
(43) Date of publication of application: 06.06.2018
(73) Proprietor: Taiki Corp., Ltd., Osaka-shi, Osaka 534-0014 (JP)
(72) Inventor: NISHIMURA, Muneto, Osaka-shi, Osaka 533-0031 (JP)
(74) Representative: Grünecker Patent- und Rechtsanwälte PartG mbB
(86) International application number: PCT/JP2016/070374
(87) International publication number: WO 2017/018181

(56) References cited:
- EP-A1- 1 374 834
- EP-A1- 2 453 047
- GB-A- 1 498 363
- JP-A- H09 220 117
- JP-A- 2001 226 225
- JP-A- 2013 192 700
- JP-A- 2015 513 987

## Description

### Technical Field

The present invention relates to a pad for transferring and supplying a liquid cosmetic preparation to a cosmetic preparation applicator by impregnating/retaining the liquid cosmetic preparation in the pad.

### Prior Art

Patent Literature 1 describes use of a polyether urethan foam having a reticulated structure, as a base material in which a liquid cosmetic preparation is impregnated/retained to be supplied to a cosmetic preparation applicator which is assembled into a cosmetic container and used for applying the liquid cosmetic preparation, wherein it is claimed that the polyether urethan foam can be sufficiently impregnated with the liquid cosmetic preparation, has excellent durability and stability after having been impregnated with the liquid cosmetic preparation, and can increase the feeling of satisfaction in using and applying the liquid cosmetic preparation.

Patent Literature 2 describes a laminated sheet comprising a liquid-retention layer comprising a non woven structural member and an adhesion layer which is laminated on at least one side of the liquid-retention layer.

### Prior Art Literature

### Patent Literature

Patent Literature 1 Japanese Patent Laid-open No. 2013-530252
Patent Literature 2 European Patent Application EP 2 453 047 A1

### Summary of the Invention

### Problems to Be Solved by the Invention

As described in the aforementioned prior art, polyether urethane foams in which a liquid cosmetic preparation is impregnated/retained so that the liquid cosmetic preparation can be transferred to a puff or other cosmetic preparation applicator and ultimately applied on the skin, are known; however, reportedly these polyether urethane foams in which a liquid cosmetic preparation is impregnated/retained do not go beyond offering excellent durability and stability, or excellent feeling of satisfaction in using and applying a liquid cosmetic preparation as it is transferred to a cosmetic preparation applicator and supplied to make up one's face. No consideration is given to their functionality in terms of how a liquid cosmetic preparation is transferred to a cosmetic preparation applicator and supplied, or specifically how the impregnated quantity of the liquid cosmetic preparation changes, how many times the cosmetic preparation can be applied using the cosmetic preparation applicator, and other details on how to reduce the swelling and deterioration of the polyether urethane foams from extended storage or the remaining quantity of the cosmetic preparation.

The object of the present invention is to provide a liquid cosmetic preparation retention/supply pad which has a large quantity of liquid cosmetic preparation impregnated/retained in it and allows as much of the impregnated/retained liquid cosmetic preparation as possible to be transferred to a cosmetic preparation applicator and supplied in a uniform, stable manner throughout the course of use of the pad from the beginning to the end.

### Means for Solving the Problems

Under the present invention, the means for solving the aforementioned problems are a liquid cosmetic preparation retention/supply pad constituted by a nonwoven fabric and a porous sheet laminated on a surface of the nonwoven fabric, characterized in that the fibres constituting the nonwoven fabric have a PP/PE sheath-core structure. In an embodiment of the invention, the porous sheet is made of an olefin resin. 3. In another embodiment of the invention, the nonwoven fabric has a density of 8,000 to 45,000 g/m³.

### Effects of the Invention

According to the present invention, a liquid cosmetic preparation can be impregnated/retained by a larger quantity, and the impregnated/retained liquid cosmetic preparation can be applied with a smaller force, and in a uniform, stable manner, on the skin using a cosmetic preparation applicator throughout the course of use of the pad from the beginning to the end. In addition, the liquid cosmetic preparation retention/supply pad does not swell or undergo much volume change even when the quantity of the impregnated liquid cosmetic preparation decreases as the pad is used from the state where the cosmetic preparation is impregnated/retained in the pad, which allows for housing of the pad in a container without fail and also leads to other benefits such as prevention of deterioration of the pad and reduction of the cosmetic preparation remaining after using the pad.

### Modes for Carrying Out the Invention

### (Nonwoven Fabric)

The nonwoven fabric used in the present invention is a nonwoven fabric made of fibers having a PP/PE sheath-core structure. Thereby, the lamination strength of the sheet can be maintained. In this PP/PE sheath-core structure, a polyethylene core is covered with a polypropylene sheath surrounding the polyethylene.

Preferably the nonwoven fabric has a density of 8,000 to 45,000 g/m³, or more preferably 10,000 to 45,000 g/m³. In addition, preferably the nonwoven fabric has a thickness of 3 to 40 mm. When the density and thickness of the nonwoven fabric are within these ranges, a liquid cosmetic preparation can be properly impregnated/retained and properly supplied to a cosmetic preparation applicator. Here, "the supply of a liquid cosmetic preparation" means that, as a cosmetic preparation applicator contacts/presses a liquid cosmetic preparation retention/supply pad in which a liquid cosmetic preparation is impregnated/retained as proposed by the present invention, the liquid cosmetic preparation is transferred to the cosmetic preparation applicator from the liquid cosmetic preparation retention/supply pad, and the liquid cosmetic preparation attaches to/is impregnated in the cosmetic preparation applicator. If the density of the nonwoven fabric is less than 8,000 g/m³, the liquid cosmetic preparation retention/supply pad does not have sufficient thickness, and its cosmetic preparation retention property tends to worsen; if the density exceeds 45,000 g/m³, on the other hand, the liquid cosmetic preparation retention/supply pad becomes too thick and its cosmetic property discharge property tends to worsen.

### (Porous Sheet)

For the porous sheet used under the present invention, any known porous sheet having through holes penetrating the sheet from its top face to bottom face, which is not a nonwoven fabric called "porous net" or "porous film," may be used. Preferably a non-water-absorbing porous sheet made of a polyester resin, (meth)acrylic resin, or olefin resin such as polyethylene resin or polypropylene resin, is used, among others. By using the pad by pressing a cosmetic preparation applicator against this porous sheet and thereby transferring a liquid cosmetic preparation to the applicator, the liquid cosmetic preparation can be applied on the skin in a uniform, stable manner, without causing fluctuation in the quantity of the liquid cosmetic preparation transferred to the cosmetic preparation applicator, throughout the course of use of the pad from the beginning to the end.

If this porous sheet is not used, the quantity of the liquid cosmetic preparation transferred to the cosmetic preparation applicator fluctuates, and the liquid cosmetic preparation tends to be supplied excessively to the cosmetic preparation applicator, especially right after the start of using the pad.

Preferably the porous sheet has a porosity of 10 to 100 mesh/inch. If the porosity is too low, supplying an appropriate quantity of the liquid cosmetic preparation from the nonwoven fabric to the cosmetic preparation applicator, as the liquid cosmetic preparation is taken up, becomes difficult. If the porosity is too high, on the other hand, the liquid cosmetic preparation is supplied by an excessive quantity all at once, which makes it difficult to make up one's face properly.

Also, preferably the porous sheet has a thickness in a range that allows an appropriate quantity of the cosmetic preparation to be supplied to the surface of the cosmetic preparation applicator. Such thickness is in a range of 1.0 to 500 µm, although the specific thickness range varies depending on the porosity.

For the lamination of such porous sheet on the surface of the nonwoven fabric, any known means relating to resin lamination can be adopted, such as any means for partial bonding or adhesion.

### (Liquid Cosmetic Preparation)

Either a water-based or oil-based liquid cosmetic preparation may be impregnated into the liquid cosmetic preparation retention/supply pad proposed by the present invention. It should be noted, however, that when the liquid cosmetic preparation is impregnated in the liquid cosmetic preparation retention/supply pad, and the pad is placed in a cosmetic container, the liquid cosmetic preparation must be transferred to a cosmetic preparation applicator from the surface of the pad and applied on the skin.

### (How to Use)

The liquid cosmetic preparation retention/supply pad proposed by the present invention may be used in virtually the same way any conventional pad, consisting of a sponge, etc., in which a liquid cosmetic preparation is absorbed, is used, such as installing it in a cosmetic container.

The porous-sheet-laminated side of the liquid cosmetic preparation retention/supply pad proposed by the present invention is pressed by the cosmetic preparation applicator so that an appropriate quantity of the liquid cosmetic preparation is transferred to the cosmetic preparation applicator.

Then, the cosmetic preparation applicator to which the liquid cosmetic preparation has been transferred, is held in contact with the skin with a certain degree of force to apply and spread the liquid cosmetic preparation to make up one's face as desired.

### Example

A liquid cosmetic preparation retention/supply pad according to the present invention, and pads according to comparative examples, were each installed in a dish-shaped container, and a liquid cosmetic preparation was impregnated/retained in each of these pads.

Next, a cosmetic preparation applicator was pressed against the surface of each pad in which the liquid cosmetic preparation had been impregnated/retained, so that the liquid cosmetic preparation is transferred to the cosmetic preparation applicator.

Then, a liquid cosmetic preparation application test was conducted by repeatedly pressing this application face of the cosmetic preparation applicator to which the liquid cosmetic preparation had been transferred, against the surface of a separately prepared nonwoven fabric.

In Tables 1 to 4 below, "nonwoven fabric" represents an example where a pad constituted only by a nonwoven fabric identical to the one used under the present invention, but on which no porous sheet was laminated, was used; "nonwoven fabric + porous sheet" represents an example where the pad proposed by the present invention was used; and "NBR" represents an example where a pad constituted by a porous sponge of synthetic rubber NBR was used.

For the nonwoven fabric, one made of fibers having a PP/PE sheath-core structure (20D, 100%, 390 g/m²) was used.

The size of each pad used in this Example, when dry, was measured. Then, from the measured size, the volume of each pad was calculated. The results are shown in Table 1.

**Table 1**

| | Pad size when dry (length x width x thickness (mm)) | Pad volume when dry (cm³) |
|---|---|---|
| Nonwoven fabric | 30 x 30 x 11.80 | 10.620 |
| Nonwoven fabric + Porous sheet | 30 x 30 x 11.25 | 10.125 |
| NBR | 30 x 30 x 7.55 | 6.795 |

Each pad was installed in a dish-shaped container (diameter 60 mm x height 5 mm), and the weight of the dish-shaped container with the pad was measured. Next, a liquid cosmetic preparation ("Integrate Mineral Watery Foundation" manufactured by Shiseido) was impregnated/retained in the pad installed in each dish-shaped container, and the weight of the dish-shaped container with the pad in which the liquid cosmetic preparation had been impregnated/retained was measured. It should be noted that, when the liquid cosmetic preparation was impregnated/retained in the pad, the liquid cosmetic preparation was gradually seeped into the pad installed in the dish-shaped container until enough liquid cosmetic preparation was impregnated/retained in the pad so that the preparation oozed out when the pad was slightly pressed.

Then, the weight of the liquid cosmetic preparation impregnated/retained in each pad was calculated from the weight of dish-shaped container with each pad and the weight of dish-shaped container with each pad in which the liquid cosmetic preparation had been impregnated/retained, after which the weight of the liquid cosmetic preparation impregnated/retained per unit volume of each pad was calculated from the weight of the liquid cosmetic preparation impregnated/retained in each pad and the volume of each pad when dry. These results are shown in Table 2.

**Table 2**

| | Weight of dish-shaped container with each pad (g) | Weight of dish-shaped container with each pad in which the liquid cosmetic preparation is impregnated/retained (g) | Weight of the liquid cosmetic preparation impregnated/retained in each pad (g) | Pad volume when dry (cm³) | Weight of the liquid cosmetic preparation impregnated/retained per unit volume of each pad (g/cm³) |
|---|---|---|---|---|---|
| Nonwoven fabric | 2.66 | 11.41 | 8.75 | 10.620 | 0.824 |
| Nonwoven fabric + Porous sheet | 2.80 | 11.80 | 9.00 | 10.125 | 0.889 |
| NBR | 3.44 | 8.07 | 4.63 | 6.795 | 0.681 |

Based on these results, the liquid cosmetic preparation was impregnated/retained in the nonwoven fabric pad by a larger quantity than in the NBR pad whose impregnation/retention quantity of the liquid cosmetic preparation had been confirmed the largest among all sponge-type pads. It was also found that the liquid cosmetic preparation was impregnated/retained by a larger quantity in the pad having the porous sheet laminated on the surface of the nonwoven fabric, compared to the pad having the nonwoven fabric only, attributed to the fact that the slight pressure applied at the time of lamination increases the retention quantity per unit volume.

These pads in which the liquid cosmetic preparation had been impregnated/retained, installed in the dish-shaped containers, were evaluated based on continuous applications using a cosmetic preparation applicator.

To be specific, the application face of the cosmetic preparation applicator was pressed once against each pad in which the liquid cosmetic preparation had been impregnated/retained so that the liquid cosmetic preparation was transferred to the application face of the cosmetic preparation applicator, after which the application face of the cosmetic preparation applicator to which the liquid cosmetic preparation had been transferred was pressed against the surface of a separately prepared nonwoven fabric with a light force. These series of operations were counted as one time, and repeated until the liquid cosmetic preparation could no longer be applied to the surface of the nonwoven fabric (end of application).

After the end of application, the weight of dish-shaped container with each pad in which the liquid cosmetic preparation remained (weight of dish-shaped container with each pad after the end of application of the liquid cosmetic preparation) was measured. Then, the weight of the liquid cosmetic preparation supplied from each pad was calculated from the weight of dish-shaped container with each pad in which the liquid cosmetic preparation had been impregnated/retained and the weight of dish-shaped container with each pad after the end of application of the liquid cosmetic preparation, after which the ratio of supply of the liquid cosmetic preparation from each pad was calculated from the weight of the liquid cosmetic preparation supplied from each pad and the weight of the liquid cosmetic preparation impregnated/retained in each pad. These results are shown in Table 3.

**Table 3**

| | Weight of dish-shaped container with each pad in which the liquid cosmetic preparation is impregnated/retained (g) | Weight of dish-shaped container with each pad after the end of application of the liquid cosmetic preparation (g) | Weight of the liquid cosmetic preparation supplied from each pad (g) | Weight of the liquid cosmetic preparation impregnated/retained in each pad (g) | Ratio of supply of the liquid cosmetic preparation from each pad (%) |
|---|---|---|---|---|---|
| Nonwoven fabric | 11.41 | 3.95 | 7.46 | 8.75 | 85.26 |
| Nonwoven fabric + Porous sheet | 11.80 | 4.28 | 7.52 | 9.00 | 83.56 |
| NBR | 8.07 | 6.02 | 2.05 | 4.63 | 44.28 |

Also, the ratio of remains of the liquid cosmetic preparation in each pad was calculated from the weight of the liquid cosmetic preparation impregnated/retained in each pad and the weight of the liquid cosmetic preparation supplied from each pad. These results are shown in Table 4.

**Table 4**

| | Weight of the liquid cosmetic preparation impregnated/retained in each pad (g) | Weight of the liquid cosmetic preparation supplied from each pad (g) | Weight of the liquid cosmetic preparation remaining in each pad (g) | Ratio of remains of the liquid cosmetic preparation in each pad (%) |
|---|---|---|---|---|
| Nonwoven fabric | 8.75 | 7.46 | 1.29 | 14.74 |
| Nonwoven fabric + Porous sheet | 9.00 | 7.52 | 1.48 | 16.44 |
| NBR | 4.63 | 2.05 | 2.58 | 55.72 |

Based on these results, the ratio of supply of the liquid cosmetic preparation was far higher, and the ratio of remains of the liquid cosmetic preparation was far lower, with the nonwoven fabric pad than with the NBR pad.

Also, the measured results regarding the number of operations until the end of application, in this experiment, are shown in Table 5.

**Table 5**

| | Number of operations until the end of application (times) |
|---|---|
| Nonwoven fabric | 271 |
| Nonwoven fabric + Porous sheet | 331 |
| NBR | 111 |

Based on these results, the nonwoven fabric pad could supply the liquid cosmetic preparation over a sustained period of time longer than the NBR pad could. It was also found that the pad having the porous sheet laminated on the surface of the nonwoven fabric pad could demonstrate excellent sustainability, uniformity, and stability of supply compared to the pad having the nonwoven fabric only, given the greater number of operations until the end of application compared to the pad having the nonwoven fabric only.

Furthermore, to check each pad for change in shape, the size of each pad after the end of application was measured, and the coefficient of linear expansion of each pad was calculated from its pad size when dry and pad size after the end of application.

**Table 6**

| | Pad size when dry (length x width x thickness (mm)) | Pad size at end of application (length x width x thickness (mm)) | Coefficient of linear expansion (%) |
|---|---|---|---|
| Nonwoven fabric | 30 x 30 x 11.80 | 30.23 x 30.59 x 10.30 | 1.37 |
| Nonwoven fabric + Porous sheet | 30 x 30 x 11.25 | 30.36 x 30.28 x 10.03 | 1.07 |
| NBR | 30 x 30 x 7.55 | 31.61 x 31.58 x 8.02 | 5.32 |

| | | | |
|---|---|---|---|
| * The coefficient of linear expansion was calculated based on the length and width. The thickness was not considered. | | | |

Based on these results, the nonwoven fabric pad had a far lower coefficient of linear expansion than the NBR pad. Also, the pad having the porous sheet laminated on the surface of the nonwoven fabric had a lower coefficient of linear expansion than the pad having the nonwoven fabric only, partly because the porous sheet regulated the swelling of the nonwoven fabric. Here, a low coefficient of linear expansion means less deformation of the pad itself, which also makes the pad excellent in that its deformation/deterioration is prevented during storage.

According to the results of the liquid cosmetic preparation application test mentioned above, the liquid cosmetic preparation retention/supply pad proposed by the present invention has higher impregnation/retention capability than sponge-type pads, as well as high liquid cosmetic preparation supply capability, with the nonwoven fabric used. Furthermore, the pad also presents such characteristics in terms of being able to supply the liquid cosmetic preparation more uniformly and stably than the pad having the nonwoven fabric only and undergoing little deformation or deterioration of the pad itself, with the porous sheet laminated on the surface of the nonwoven fabric.

This means that the liquid cosmetic preparation retention/supply pad proposed by the present invention is useful, as a base material to be installed in a cosmetic container, etc., in which a liquid cosmetic preparation is impregnated/retained, in that the pad can supply the cosmetic preparation by a larger quantity in a stable manner, does not swell or deteriorate due to storage, and can be retained in the cosmetic container without fail.

## Claims

1. A liquid cosmetic preparation retention/supply pad constituted by a nonwoven fabric and a porous sheet laminated on a surface of the nonwoven fabric, **characterized in that** the fibers constituting the nonwoven fabric have a PP/PE sheath-core structure.

2. The liquid cosmetic preparation retention/supply pad according to Claim 1, wherein the porous sheet is made of an olefin resin.

3. The liquid cosmetic preparation retention/supply pad according to Claim 1 or 2, wherein the nonwoven fabric has a density of 8,000 to 45,000 g/m³.

## Patentansprüche

1. Pad zur Rückhaltung/Bereitstellung einer flüssigen Kosmetikzubereitung, gebildet durch einen Vliesstoff und eine poröse Schicht, die auf eine Oberfläche des Vliesstoffs laminiert ist, **dadurch gekennzeichnet, dass** die Fasern, die den Vliesstoff bilden, eine PP/PE Schale-Kern-Struktur aufweisen.

2. Pad zur Rückhaltung/Bereitstellung einer flüssigen Kosmetikzubereitung gemäß Anspruch 1, wobei die poröse Schicht aus einem Olefinharz hergestellt ist.

3. Pad zur Rückhaltung/Bereitstellung einer flüssigen Kosmetikzubereitung gemäß Anspruch 1 oder 2, wobei der Vliesstoff eine Dichte von 8.000 bis 45.000 g/cm³ aufweist.

## Revendications

1. Tampon de rétention/alimentation de préparation cosmétique liquide constitué d'un textile non-tissé et d'une feuille poreuse stratifiée sur une surface du textile non-tissé, **caractérisé en ce que** les fibres constituant le textile non-tissé ont une structure de gaine en PP/PE-cœur.

2. Tampon de rétention/alimentation de préparation cosmétique liquide selon la revendication 1, la feuille poreuse étant constituée d'une résine d'oléfine.

3. Tampon de rétention/alimentation de préparation cosmétique liquide selon la revendication 1 ou 2, le textile non-tissé ayant une densité de 8 000 à 45 000 g/m³.
